# EUROPÄISCHE PATENTSCHRIFT

(11) **EP 1 230 265 B1**
(45) Veröffentlichungstag und Bekanntmachung des Hinweises auf die Patenterteilung: **13.06.2007**
(21) Anmeldenummer: 00983011.8
(22) Anmeldetag: 13.10.2000
(51) Int. Cl.: C07K 14/435, G01N 33/574, C12Q 1/68

(54) **NUKLEINSÄURE, DIE FÜR EIN PROTEIN DER CTAGE-FAMILIE KODIERT, DEREN VERWENDUNG UND HERSTELLUNG**
CTAGE GENE FAMILY
FAMILLE GENIQUE POUR CTAGE

(30) Priorität: 14.10.1999 DE 19949595
(43) Veröffentlichungstag der Anmeldung: 14.08.2002
(73) Patentinhaber: Deutsches Krebsforschungszentrum Stiftung des öffentlichen Rechts, 69120 Heidelberg (DE)
(72) Erfinder: EICHMÜLLER, Stefan, 68535 Edingen-Neckarhausen (DE); SCHADENDORF, Dirk, 68165 Mannheim (DE); USENER, Dirk, 64289 Darmstadt (DE)
(74) Vertreter: Schüssler, Andrea
(86) Internationale Anmeldenummer: PCT/DE2000/003628
(87) Internationale Veröffentlichungsnummer: WO 2001/027255

(56) Entgegenhaltungen:
- WO-A-99/54738
- US-A- 5 804 381
- HECKEL DIRK ET AL: "cDNA cloning and chromosomal mapping of a predicted coiled-coil proline-rich protein immunogenic in meningioma patients." HUMAN MOLECULAR GENETICS, Bd. 6, Nr. 12, November 1997 (1997-11), Seiten 2031-2041, XP000938615 ISSN: 0964-6906
- DATABASE EMBL:HSU73682 [Online] AC:U73682, 3. April 1997 (1997-04-03) HECKEL D ET AL.: "Human meningioma-expressed antigen 11 8MEA11) mRNA" XP002163598
- "EXPRESSION OF MULTIPLE CANCER/TESTIS (CT) ANTIGENS IN BREAST CANCER AND MELANOMA: BASIS FOR POLYVALENT CT VACCINE STRATEGIES" INTERNATIONAL JOURNAL OF CANCER,NEW YORK, NY,US, Bd. 78, Nr. 3, 1998, Seiten 387-389, XP000916550 ISSN: 0020-7136
- SAHIN UGUR ET AL: "Serological identification of human tumor antigens." CURRENT OPINION IN IMMUNOLOGY, Bd. 9, Nr. 5, Oktober 1997 (1997-10), Seiten 709-716, XP000985630 ISSN: 0952-7915
- HECKEL DIRK ET AL: "Novel immunogenic antigen homologous to hyaluronidase in meningioma." HUMAN MOLECULAR GENETICS, Bd. 7, Nr. 12, November 1998 (1998-11), XP002163597 ISSN: 0964-6906
- L. FRANK GLASS ET AL.: "Cutaneous T-cell Lymphoma" CANCER CONTROL, Bd. 5, Nr. 1, Januar 1998 (1998-01), Seiten 11-18, XP000985657
- JAEGER E ET AL: "HUMORAL IMMUNE RESPONSES OF CANCER PATIENTS AGAINST CANCER-TESTIS ANTIGEN NY-ESO-1: CORRELATION WITH CLINICAL EVENTS" INTERNATIONAL JOURNAL OF CANCER,NEW YORK, NY,US, Bd. 84, Nr. 5, 22. Oktober 1999 (1999-10-22), Seiten 506-510, XP000964729 ISSN: 0020-7136
- ZENDMAN A J W ET AL: "CTP11, A NOVEL MEMBER OF THE FAMILY OF HUMAN CANCER/TESTIS ANTIGENS" CANCER RESEARCH,AMERICAN ASSOCIATION FOR CANCER RESEARCH, BALTIMORE, MD,US, Bd. 59, Nr. 24, 1999, Seiten 6223-6229, XP000919167 ISSN: 0008-5472
- EICHMUELLER STEFAN ET AL: "Serological detection of cutaneous T-cell lymphoma-associated antigens." PROCEEDINGS OF THE NATIONAL ACADEMY OF SCIENCES OF THE UNITED STATES, Bd. 98, Nr. 2, 16. Januar 2001 (2001-01-16), Seiten 629-634, XP000985313 January 16, 2001 ISSN: 0027-8424

## Beschreibung

Die vorliegende Erfindung betrifft eine neue Genfamilie für "cutaneous T-cell lymphoma associated genes" (CTAGE). Die vorliegende Erfindung beschreibt zwei Mitglieder dieser Familie, CTAGE-1 und CTAGE-2, deren zugrundeliegende cDNA (ctage-1 und ctage-2) und deren Einsatz für Diagnose und Therapie von Tumorerkrankungen.

Das kutane T-Zell-Lymphom (CTCL) ist ein von T-Lymphozyten ausgehender Tumor der Haut, der im fortgeschrittenen Stadium nur schwer zu behandeln ist Die kutanen T-Zell-Lymphome zählen allgemein zur Gruppe der Non-Hodgkin-Lymphomen. Zu den kutanen T-Zell-Lymphomen gehören u.a. die Erkrankungen Mycosis fungoides, das Sézary-Syndrom und die pagetoide Retikulose. Konventionelle Therapien (Bestrahlung, Chemotherapie) sind im metastasierenden Krankheitsstadium bzw. als vorbeugende Therapie nach Entfernung des Primärtumors häufig nur schlecht wirksam und zudem mit den bekannten starken Nebenwirkungen behaftet. Oft ist es sogar so, daß sich der Primärtumor so weit ausgedehnt hat und so gestreut hat, daß eine operative Therapie keinen Sinn hat. In letzter Zeit wurde gerade für Hauttumoren die Möglichkeit einer möglichen Immuntherapie (Impfungen gegen Tumoren) in Erwägung gezogen. Die Anwendung solcher Therapieformen bedarf aber Tumor-spezifischer Antigene, die bislang für viele Tumorarten nicht gefunden wurden.

Tumor-spezifische Antigene, die neben Tumorgewebe auch in Testis-Gewebe nachgewiesen werden konnten, werden beschrieben in "Expression of multiple cancer/testis (CT) antigens in breast cancer and melanoma: basis for polyvalent CT vaccine strategies", International Journal of Cancer, Bd. 78, Nr. 3, 1998, Seiten 387-389; US 5 804 381 sowie Sahin U. et al:"Serological identification of human tumor antigens", Current Opinion in Immunology, Bd. 9, Nr. 5, 1997, Seiten 709-716.

Somit liegt der Erfindung im Wesentlichen das technische Problem zugrunde, für kutane T-Zell-Lymphome geeignete tumor-spezifische Antigene zu finden, die im Rahmen einer Vakzinierungstherapie verwendet werden können.

Die Lösung dieses technischen Problems wurde durch die Bereitstellung der in den Patentansprüchen gekennzeichneten Ausführungsformen erreicht.

Von den Erfindern konnte eine Familie von DNA-Molekülen isoliert werden, die außer in Testis-Gewebe nur in verschiedenen Tumorgeweben, insbesondere bei bei T-Zell-Lymphomen (wie dem Sézary-Syndrom), Tumoren des HNO-Bereichs oder Ovarial-Karzinomen, exprimiert wird (s. Beispiele 4 und 5). Sie gehören zu den sogenannten "Cancer-Testis-Antigenen". Die Identifizierung solcher Gene (CTAGE-1 und CTAGE-2) sind von Interesse, da die davon kodierten Proteine und von diesen abgeleitete Peptide als Zielstrukturen, z.B. für zytotoxische Zellen, dienen und als Antigene zur Produktion von diagnostischen oder therapeutischen Antikörpern verwendet werden können. Zur Tumortherapie können die von CTAGE-1 bzw. CTAGE-2 kodierten Peptide bzw. Fragmente davon entweder direkt appliziert werden oder auf Antigen-präsentierende Zellen geladen werden. Auch können die Antigene darstellenden Peptide mit Hilfe von Vektoren in verschiedenen Zellen (z.B. Dendritischen Zellen als Antigen-präsentierende Zellen) exprimiert werden. Weiter bildet die Klonierung von einem oder mehreren Vertretern der CTAGE-Genfamilie die Grundlage zur Entwicklung diagnostischer Tests, um zukünftig eine zuverlässigere und frühzeitige Diagnose bei Betroffenen zu gewährleisten. Darüberhinaus werden funktionelle Analysen des Proteins zweifellos zum Verständnis der Tumorentwicklung beitragen. Sie sind somit als Kandidatengene für Untersuchungen der Pathomechanismen anzusehen, die verschiedenen Tumorerkrankungen zugrunde liegen.

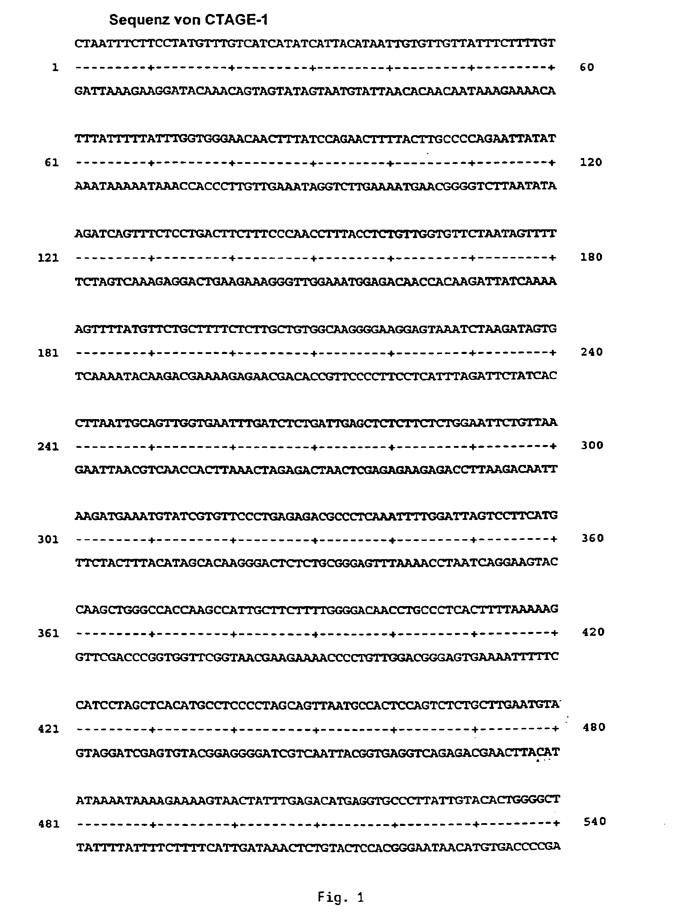

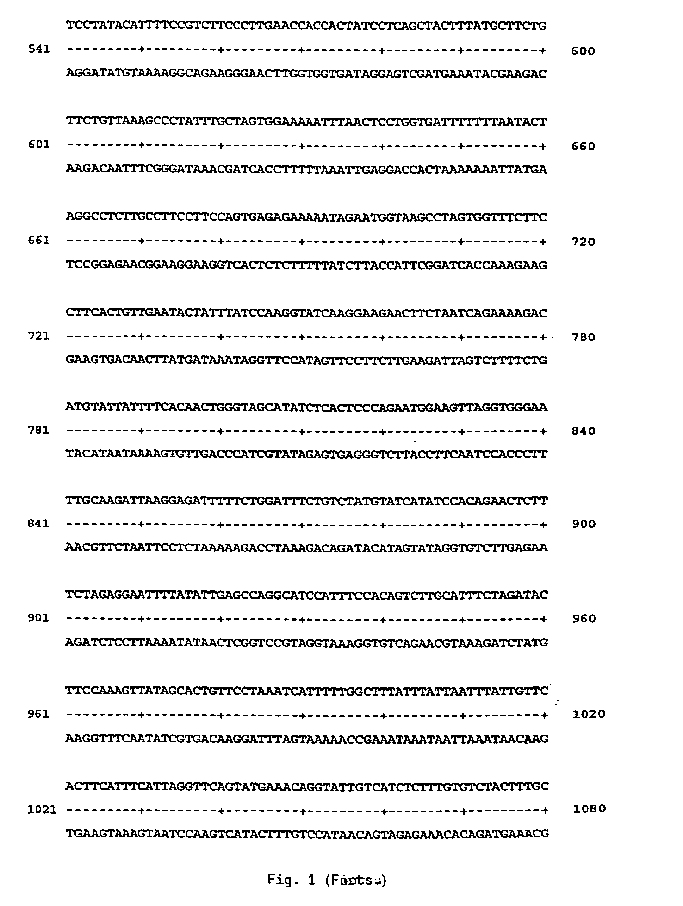

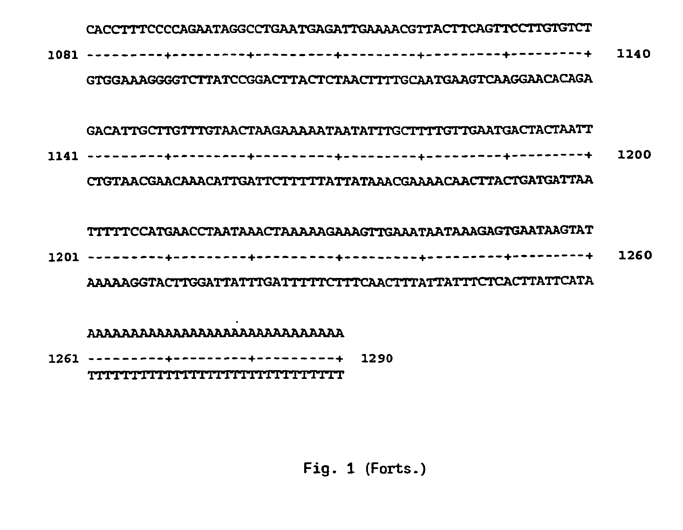

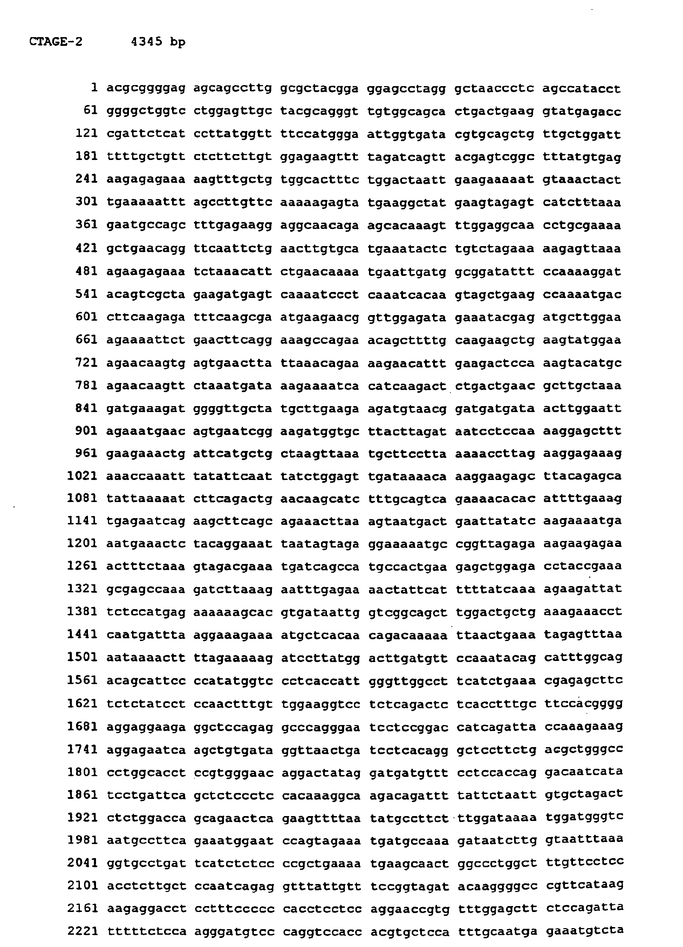

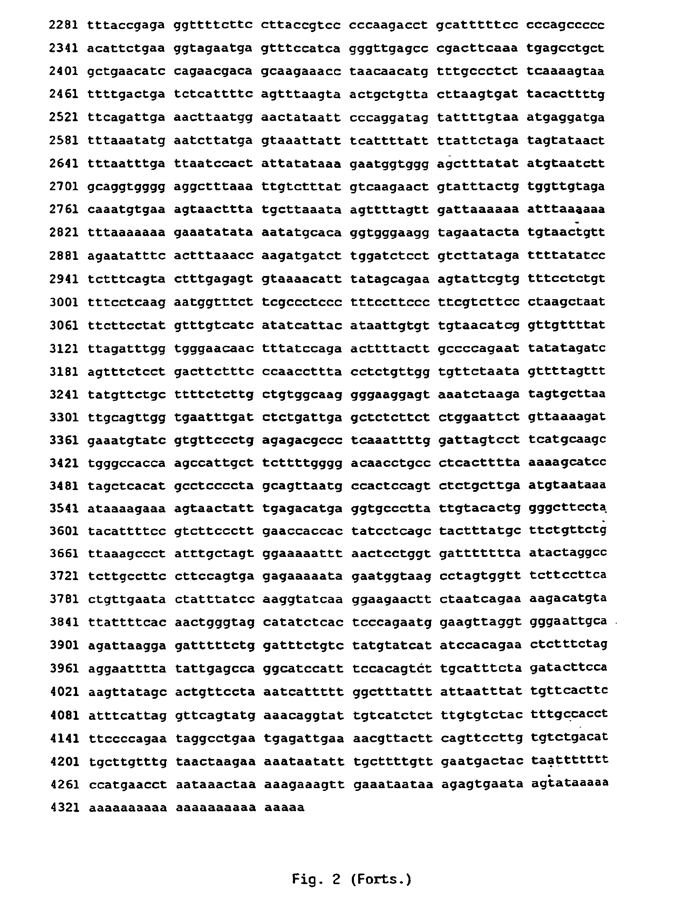

Somit betrifft die vorliegende Erfindung ein DNA-Molekül codierend für ein tumor-assoziiertes Antigen, das besteht aus:
(a) der Nukleinsäure von Fig. 1 oder Fig. 2, oder einer dazu mindestens 80% identischen Nukleinsäure,
(b) einer mit der Nucleinsäure von (a) über den degenerierten genetischen Code verwandten DNA.

Die unter (a) und (b) definierten Nucleinsäuremoleküle codieren für Proteine, Polypeptide oder Peptide, die mindestens noch eine der nachstehend beschriebenen biologischen Aktivitäten des von der Nukleinsäure gemäß Fig. 1 oder Fig. 2 codierten Proteins aufweisen, beispielsweise ein tumor-spezifisches Antigen darstellen.

Die unter (a) definierten DNA-Moleküle umfassen auch DNA-Moleküle, die sich gegenüber der in Figur 1 bzw. 2 angegebenen Sequenz durch Deletion(en), Insertion(en), Austausch(e) und/oder andere im Stand der Technik bekannte Modifikationen, z.B. alternatives Splicing, unterscheiden bzw. ein Fragment des ursprünglichen Nucleinsäuremoleküls umfassen, wobei das durch diese DNA-Moleküle codierte Protein noch eine oder mehrere der vorstehend beschriebenen biologischen Aktivitäten aufweist. Dazu zählen auch Allelvarianten. Verfahren zur Erzeugung der vorstehenden Änderungen in der Nucleinsäuresequenz sind dem Fachmann bekannt und in Standardwerken der Molekularbiologie beschrieben, beispielsweise in Sambrook et al., Molecular Cloning: A Laboratory Manual, 2. Ausgabe, Cold Spring Harbor Laboratory Press, Cold Spring Harbor NY (1989). Die Varianten haben dabei mindestens 80%, bevorzugt 90, ganz bevorzugt 95, 96, 97, 98 oder 99% Identität zu den Sequenzen gemäß Fig. 1 oder 2.

In einer besonders bevorzugten Ausführungsform ist das erfindungsgemäße DNA-Molekül eine cDNA.

In einer weiteren bevorzugten Ausführungsform ist das erfindungsgemäße DNA-Molekül eine genomische DNA, die vorzugsweise von einem Säuger, beispielsweise einem Menschen stammt. Auf Nucleinsäurehybridisierung basierende Screening-Verfahren erlauben die Isolierung der erfindungsgemäßen genomischen DNA-Moleküle aus jedem Organismus bzw. abgeleiteten genomischen DNA-Banken, wobei Sonden verwendet werden, die die in Figur 1 oder 2 angegebene Nucleinsäuresequenz oder einen Teil davon enthalten.

Eine erfindungsgemäße Nukleinsäure eignet sich besonders als Antigen-codierende Struktur für therapeutische Zwecke. Ziel soll es dabei sein, das Immunsystem zu stimulieren, Tumorzellen zu eliminieren, die über ein Mitglied der CTAGE-Familie, insbesondere CTAGE-1 oder CTAGE-2, identifiziert werden. Dabei gibt es verschiedene Wege, z.B. Injektion der nackten DNA in den Patienten. Hierzu wird ein Plasmid mit einem sehr aktiven Promotor und einem Mitglied der CTAGE-Familie, insbesondere CTAGE-1 oder CTAGE-2, beispielsweise in den Muskel oder intradermal injiziert. Das Ziel ist, daß die Zellen das Plasmid aufnehmen, Antigene produzieren, über HLA-Moleküle einzelne Peptide präsentieren und somit eine zytotoxische T-Zell-Immunantwort hervorrufen. Diese soll dann zur Abwehr von Tumorzellen führen. Allgemein ist diese Verfahrensweise in Conry et al., Clinical Cancer Research, Vol. 4, S. 2903-2912 (1998) beschrieben. Einen alternativen Weg stellt die "Gene-Gun"-Methode dar, die in Fynan et al., Proc. Natl. Acad. Sci. USA, Vol. 90, S. 11478-11482 (1993) beschreiben ist.

Die erfindungsgemäßen Nukleinsäuren können auch in einen Vektor bzw. Expressionsvektor inseriert werden. Somit umfaßt die vorliegende Erfindung auch diese Nucleinsäuremoleküle enthaltende Vektoren. Beispiele solcher sind dem Fachmann bekannt. Im Falle eines Expressionsvektors für E. coli sind dies z.B. pGEMEX, pUC-Derivate (z.B. pUC8), pBR322, pBlueScript, pGEX-2T, pET3b und pQE-8. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A. In einer bevorzugten Ausführungsform ist das erfindungsgemäße Nucleinsäuremolekül im Vektor mit regulatorischen Elementen funktionell verknüpft, die dessen Expression in prokaryotischen oder eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Prokaryonten, beispielsweise E.coli, zählen der lac-,trp-Promotor oder T7-Promotor, und für die Expression in Eukaryonten der AOX1- oder GAL1-Promotor in Hefe, und der CMV-, SV40-, RVS-40-Promotor, CMV- oder SV40-Enhancer für die Expression in tierischen Zellen. Weitere Beispiele für geeignete Promotoren sind der Metallothionein I- und der Polyhedrin-Promotor.

Zu geeigneten Vektoren zählen insbesondere auch T7 basierende Expressionsvektoren für die Expression in Bakterien (Rosenberg et al., Gene 56(1987), 125) oder pMSXND für die Expression in Säugerzellen (Lee und Nathans, J.Biol.Chem. 263 (1988),3521).

Desweiteren kann die erfindungsgemäße DNA nicht nur zu Zwecken der rekombinanten Herstellung in einen Vektor inseriert sein, sondern auch um die DNA mit Hilfe von Vektoren in Patienten zu injizieren, wo diese ein Antigen für therapeutische Zwecke codiert. Hierbei wird die DNA mit Hilfe eines Vektors in vivo Antigenpräsentierende Zellen (APCs), z.B. Dendritische Zellen, zur HLA-Präsentation von CTAGE gebracht. Dabei kann der die erfindungsgemäße DNA enthaltende Vektor über verschiedene Wege injiziert werden:
a) Lipid- bzw. Liposomen-verpackte DNA oder RNA, z.B. allgemein beschrieben von Nabel et al., Proc. Natl. Acad. Sci. USA, Vol. 93, S. 15388-15393 (1996)
b) Mit einem Bakterium als Transportvehikel für den Expressionsvektor. Geeignete Bakterien sind beispielsweise (attentuierte) Listerien [z.B. Listeria monocytogenes], Salmonellen-Stämme [z.B. Salmonella spp.]. Allgemein ist diese Technik von Medina et al., Eur. J. Immunol., 29, S. 693-699 (1999) sowie Guzman et al., Eur. J. Immunol. 28, S. 1807-1814 (1998) beschrieben worden. Desweiteren wird auf WO 96/14087; Weiskirch et al., Immunological Reviews, Vo. 158, S. 159-169 (1997) und US-A-5,830,702 verwiesen.
c) mittels Gene-Gun (Williams et al. Proc. Natl. Acad. Sci. USA, Vol. 88, S. 2726-2730, 1991).

In einer bevorzugten Ausführungsform ist der die erfindungsgemäßen DNA-Moleküle enthaltene Vektor ein Virus, beispielsweise ein Adenovirus, Vaccinia-Virus oder ein AAV-Virus, der bei einer Gentherapie von Nutzen ist. Besonders bevorzugt sind Retroviren. Beispiele für geeignete Retroviren sind MoMuLV, HaMuSV, MuMTV, RSV oder GaLV. Weiter eignen sich vorgenannte Viren sowie Fowlpox-Virus, Canarypox-Virus, Influenza-virus oder Sindbis-Virus auch als Basis einer Vakzine. Solche neuen Vakzine, die nach Verabreichung an den Patienten anti-Tumor-Immunität verleihen, sind beispielsweise bei N. Restifo, Current Opinion in Immunology, 8, S. 658-663 (1996) oder Ying et al., Nature Medicine, Vol. 5, Nr. 7, S. 823 ff., (1999) beschrieben. Für Zwecke der Gentherapie können die erfindungsgemäßen DNA-Moleküle auch in Form von kolloidalen Dispersionen zu den Zielzellen transportiert werden. Dazu zählen beispielsweise Liposomen oder Lipoplexe (Mannino et al., Biotechniques 6 (1988), 682).

Allgemeine auf dem Fachgebiet bekannte Verfahren können zur Konstruktion von Vektoren oder Plasmiden, die die erfindungsgemäßen DNA-Moleküle und geeignete Kontrollsequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., supra, beschrieben sind.

Die vorliegende Erfindung betrifft auch die vorstehend beschriebenen Vektoren enthaltende Wirtszellen. Zu diesen Wirtszellen zählen Bakterien, Hefe, Insekten- und Tierzellen, vorzugsweise Säugerzellen. Bevorzugt sind die E. coli Stämme HB101, DH1, x1776, JM101, JM109, BL21, XL1Blue und SG 13009, der Hefestamm Saccharomyces cerevisiae und die tierischen Zellen L, 3T3, FM3A, CHO, COS, Vero, HeLa sowie die Insektenzellen sf9. Verfahren zur Transformation dieser Wirtszellen, zur phänotypischen Selektion von Transformanten und zur Expression der erfindungsgemäßen DNA-Moleküle unter Verwendung der vorstehend beschriebenen Vektoren sind auf dem Fachgebiet bekannt.

Weiter ist es bevorzugt, um eine Tumor-Immunität zu erzeugen, die erfindungsgemäße DNA in antigenpräsentierende Zellen zu transfizieren und diese dem Patienten zu injizieren. Hierbei wird ein Plasmid in vitro in eine Antigen-präsentierende Zelle (APCs), z.B. Dendritische Zellen, gegeben, die dann Antigene produzieren und über HLA-Moleküle einzelne Peptide präsentieren. Dabei kann die Plasmid-DNA über verschiedene Wege in die Antigen-präsentierenden Zellen gebracht werden:
(a) als nackte DNA, z.B. mittels "Gene Gun" oder Elektroporation
(b) Lipid- oder Liposomen-verpackte DNA oder RNA (Nair et al., Nature Biotechnology Vol. 16, S. 364 ff. (1998))
(c) mit einem Virus als Vektor (Kim et al., J. of Immunotherapy, 20(4), S. 276-286 (1997))
(d) mit einem Bakterium als Transportvehikel für den Expressionsvektor (Medina et al., Eur. J. Immunol., 29, S. 693-699 (1999); Guzman et al., Eur. J. Immunol. 28, S. 1807-1814 (1998))

Ein Klon, der für eine erfindungsgemäße Nukleinsäure (CTAGE-1) kodiert, wurde am 7. Oktober 1999 bei der DSMZ, Deutsche Sammlung von Mikroorganismen und Zellkulturen GmbH, Mascheroder Weg 1b, 38124 Braunschweig nach dem Budapester Vertrag unter der Hinterlegungsnummer DSM 13079 (= Escherichia coli XL2-Blue pCTAGE-1) hinterlegt.

Die vorliegende Erfindung betrifft auch ein Verfahren zur Herstellung eines Proteins, das durch obige Nukleinsäuren kodiert wird, umfassend die Kultivierung der vorstehend beschriebenen Wirtszellen unter Bedingungen, die die Expression des Proteins erlauben (vorzugsweise stabile Expression), und Gewinnung des Proteins aus der Kultur. Geeignete Verfahren zur rekombinanten Herstellung des Proteins sind allgemein bekannt (siehe beispielsweise Holmgren, Annu.Rev.Biochem. 54 (1985), 237; LaVallie et al., Bio/Technology 11 (1993),187; Wong, Curr.Opin.Biotech.6 (1995), 517; Romanos, Curr.Opin.Biotech.6 (1995), 527; Williams et al., Curr. Opin. Biotech.6 (1995), 538; und Davies, Curr. Opin.Biotech.6 (1995), 543. Auch geeignete Reinigungsverfahren (beispielsweise präparative Chromatographie, Affinitätschromatographie, beispielsweise Immunoaffinitätschromatographie, HPLC etc.) sind allgemein bekannt.

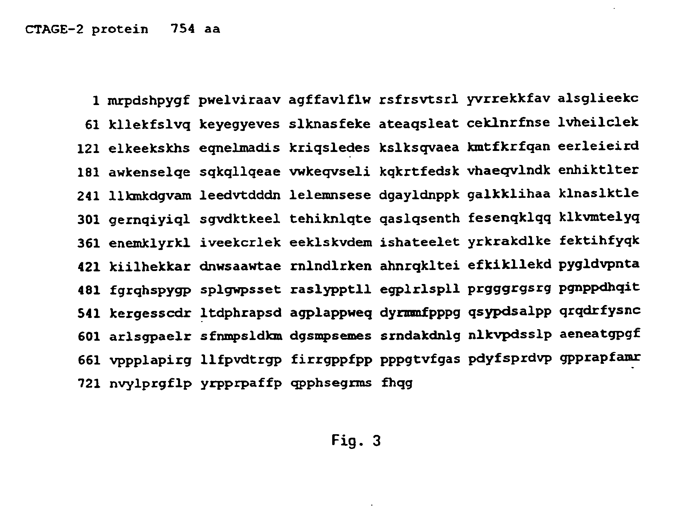

In einer weiteren Ausführungsform betrifft die vorliegende Erfindung ein von den erfindungsgemäßen DNA-Molekülen (CTAGE-1 oder CTAGE-2) codiertes oder nach dem vorstehenden Verfahren erhaltenes Protein. Das für CTAGE-2 codierende Protein ist in Fig. 3 gezeigt. In diesem Zusammenhang soll erwähnt werden, daß das erfindungsgemäße Protein gemäß üblicher, auf dem Fachgebiet bekannten, Verfahren modifiziert sein kann. Zu diesen Modifikationen zählen Austausche, Insertionen oder Deletionen von Aminosäuren, die die Struktur des Proteins modifizieren, wobei seine biologische Aktivität im wesentlichen erhalten bleibt. Zu den Austauschen zählen vorzugsweise "konservative" Austausche von Aminosäureresten, d.h. Austausche gegen biologisch ähnliche Reste, z.B. die Substitution eines hydrophoben Rests (z.B. Isoleucin, Valin, Leucin, Methionin) gegen einen anderen hydrophoben Rest, oder die Substitution eines polaren Rests gegen einen anderen polaren Rest (z.B. Arginin gegen Lysin, Glutaminsäure gegen Asparaginsäure etc.). Deletionen können zur Erzeugung von Molekülen führen, die eine deutlich geringere Größe aufweisen, d.h., denen beispielsweise Aminosäuren am N- oder C-Terminus fehlen. Die Varianten haben dabei mindestens 80%, bevorzugt 90, ganz bevorzugt 95, 96, 97, 98 oder 99% Identität zu der Aminosäuresequenz, die der Nukleotidsequenz gemäß Fig. 1 abgeleitet ist oder zu der Aminosäuresequenz gemäß Fig. 3.

Für die gewünschte Anti-Tumor-Vakzinierung eignen sich auch Injektionen des Proteins oder eines oder mehrerer davon abgeleiteter Peptide. Aus der Sequenz des erfindungsgemäßen Proteins werden dafür entweder mittels entsprechender Computerprogramme oder mittels Experimente (z.B. phagozytotische Aufnahme des Gesamt-Proteins, danach Analyse der präsentierenden Peptide) HLA-abhängige Peptid-Fragmente ermittelt. Diese werden mittels dem Fachmann bekannter Methoden künstlich hergestellt und dann (ggf. mit Immunsystem stimulierenden Faktoren, z.B. Interteronen, Interleukinen usw.) dem Patienten injiziert. Das hinter dieser Behandlung steckende Ziel ist, daß die APCs die Peptide aufnehmen, sie präsentieren und so in vivo die Produktion von Tumor-spezifischen zytotoxischen T-Zellen stimmulieren. Allgemein ist dieses Prinzip von Melief et al., Current Opinion in Immunology, 8, S. 651-657 (1996) beschrieben worden.

Ebenso wie vorstehend beschrieben, können statt des Vektors auch das erfindungsgemäße Protein bzw. Fragmente davon in vitro auf APCs geladen werden. Die beladenen Zellen werden dann dem Patienten z.B. in die Lymphknoten injiziert und sorgen direkt für die Stimmulierung und Vermehrung von Tumor-spezifischen zytotoxischen T-Zellen (Nestle et al., Nature Medicine, Vol.4, Nr. 3, S. 328 ff. (1998); Schadendorf et al., in: Burg, Dummer, Strategies for Immunointerventions in Dermatology, Springer Verlag, Berlin Heidelberg, S. 399-409, 1997) Für die Vakzinierung kann es vorteilhaft sein, gegenüber dem Wildtyp-Antigen einzelne Aminosäuren, wie vorstehend beschrieben, zu modifizieren, da damit u.U. eine Erhöhung der Bindung und eine Verbesserung der Wirksamkeit zu erreichen ist (Clay et al., The Journal of Immunology 162: S. 1749-1755, 1999).

Die vorliegende Erfindung betrifft auch Antikörper, die das vorstehend beschriebene Protein CTAGE-1 oder CTAGE-2 spezifisch erkennen. Die Antikörper können monoclonale, polyclonale oder synthetische Antikörper sein oder Fragmente davon, beispielsweise Fab-, Fv-oder scFv-Fragmente. Vorzugsweise handelt es sich dabei um monoclonale Antikörper. Für die Herstellung ist es günstig, Tiere, insbesondere Kaninchen oder Hühner für einen polyclonalen und Mäuse für einen monoclonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyclonale Antikörper kann dann aus dem Serum bzw. Eigelb der Tiere erhalten werden. Die erfindungsgemäßen Antikörper können gemäß Standardverfahren hergestellt werden, wobei das von den erfindungsgemäßen DNA-Molekülen codierte Protein oder ein synthetisches Fragment davon als Immunogen dienen. Monoclonale Antikörper können beispielsweise durch das von Köhler und Milstein (Nature 256 (1975), 495) und Galfré (Meth. Enzymol.73 (1981), 3) beschriebene Verfahren hergestellt werden, wobei Maus-Myelomzellen mit von immunisierten Säugern stammenden Milzzellen fusioniert werden. Diese Antikörper können beispielsweise zur Immunpräzipitation der vorstehend diskutierten Proteine oder zur Isolierung verwandter Proteine aus cDNA-Expressionsbanken verwendet werden. Die Antikörper können beispielsweise in Immunoassays in Flüssigphase oder an einen festen Träger gebunden werden. Dabei können die Antikörper auf verschiedene Art und Weise markiert sein. Geeignete Marker und Markierungsverfahren sind dem Fachgebiet bekannt. Beispiele für Immunassays sind ELISA und RIA. Weiter können die Antikörper neben ihrer diagnostischen Eignung auch therapeutisch eingesetzt werden. Dabei dient z.B. ein Protein der CTAGE-Familie, z.B. CTAGE-1 oder CTAGE-2, als Target für bispezifische Antikörper. Hierzu wird auf Kastenbauer et al., Laryngorhinootologie, 78(1), S. 31-35 (1999) und Cao et al., Bioconj. Chem. 9(6), S. 635-644 (1998) verwiesen. Weiter kann durch die Gabe der Antikörper der Tumor-Wachstum und -Metastasierung begünstigenden Wirkung der CTAGE-Proteine entgegengewirkt werden.

Desweiteren kann durch den Einsatz von CTAGE-1 oder CTAGE-2 Antisense DNA eine Inhibierung der Translation von CTAGE-1 oder CTAGE-2 erreicht werden und somit ein therapeutischer Effekt spezifisch auf dieses Gen ausgeübt werden. Es bilden sich in den entsprechenden Tumorzellen RNA/DNA-Hybride, die so die Transkription verhindern und gleichzeitig einen Abbau der Hybride (und somit der RNA) durch RNase H bewirken (Scanlon et al., The Faseb Journal, Vol. 9, S. 1288-1296, 1995)

Die vorliegende Erfindung betrifft ferner die Verwendung der vorstehend beschriebenen DNA-Moleküle, Vektoren, Proteine und/oder Antikörper. Vorzugsweise werden diese zur Herstellung eines Arzneimittels zur Diagnose oder Behandlung von Tumorerkrankungen, in denen CTAGE-1 oder CTAGE-2 eine Rolle spielt, verwendet. Bevorzugt ist die Bereitstellung eines Vakzinierungsmittels, das wie vorstehend beschrieben, entweder auf der DNA oder dem Protein/Peptid basiert.

Diese Arzneimittel enthalten gegebenenfalls zusätzlich einen pharmazeutisch verträglichen Träger. Geeignete Träger und die Formulierung derartiger Arzneimittel sind dem Fachmann bekannt. Zu geeigneten Trägem zählen beispielsweise Phosphat-gepufferte Kochsalzlösungen, Wasser, Emulsionen, beispielsweise ÖI/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intra-arterielle, intramuskuläre, subkutane, intramedulläre, intrathekale, intraventrikuläre, intravenöse, intraperitoneale oder intranasale Verabreichung. Die geeignete Dosierung wird von dem behandelnden Arzt bestimmt und hängt von verschiedenen Faktoren ab, beispielsweise von dem Alter, dem Geschlecht, dem Gewicht des Patienten, dem Stadium der Erkrankung, der Art der Verabreichung etc.

Die vorliegende Erfindung betrifft ferner eine diagnostische Zusammensetzung, die das vorstehend beschriebene DNA-Molekül oder den Antikörper enthält oder Kombinationen davon, gegebenenfalls zusammen mit einem geeigneten Nachweismittel. Die diagnostische Zusammensetzung eignet sich dabei einerseits um eine Tumorerkrankung festzustellen, aber auch um eine Verlaufskontrolle durchzuführen.

Das erfindungsgemäße, wie vorstehend definierte DNA-Molekül kann auch als Sonde verwendet werden, um DNA-Moleküle zu isolieren, die beispielsweise von einer anderen Spezies oder einem anderen Organismus stammen und ein Protein codieren mit einer ebensolchen biologischen Aktivität. Vorzugsweise weist dazu die Sonde eine Länge von mindestens 10, besonders bevorzugt mindestens 15 Basen auf. Geeignete, auf Hybridisierung basierende Nachweisverfahren sind dem Fachmann bekannt, z.B. Southern oder Northern Blot. Geeignete Markierungen für die Sonde sind dem Fachmann ebenfalls bekannt und dazu zählen beispielsweise Markierung mit Radioisotopen, Biolumineszenz-, Chemilumineszenz-, Fluoreszenzmarkern, Metallchelaten, Enzymen etc..

Darüber hinaus kann dies auch durch eine PCR (Wiedmann et al., PCR Methods Appl. 3, S. 551-564 (1994); Saiki et al., Nature 324, S. 163-166 (1986)) oder "Ligase chain reaction" (LCR) (Taylor et al., Curr. Opin. Biotechnol. 6, S. 24-29 (1995); Rouwendal et al., Methods Mol. Biol. S. 149-156 (1996)) erfolgen, wobei die Primer von der Sequenz in Figur 1 oder Figur 2 abgeleitet sind und wobei geeignete Primer (hinsichtlich Länge, Komplementarität zur Matritze, dem zu amplifizierenden Bereich etc.) vom Fachmann gemäß üblicher Verfahren entworfen werden können.

Außerdem betrifft die vorliegende Erfindung ein Verfahren zur Diagnose von Tumorerkrankungen, in vitro, die folgenden Schritte umfassend:
- Isolierung von Nucleinsäure aus dem Patienten,
- Durchführung einer LCR oder PCR mit geeigneten Primern oder einer Hybridisierungsanalyse mit einer oder mehreren geeigneten Sonden basierend auf der codierenden Sequenz von Fig. 1 oder Fig. 2,
- Nachweis eines amplifizierten Produkts oder einer Hybridisierung als Indiz auf das Vorliegen einer Tumorerkrankung.

Bei oben genanntem Verfahren können dem Fachmann bekannte Methoden bezüglich der Präparation von DNA oder RNA aus biologischen Proben, des Restriktionsverdaus der DNA, der Auftrennung der Restriktionsfragmente auf nach Größe auftrennenden Gelen, beispielsweise Agarosegelen, der Herstellung und Markierung der Sonde und des Nachweises der Hybridisierung, beispielsweise über "Southern-Blot" oder in-situ Hybridisierung, angewandt werden.

Der vorstehende Nachweis kann auch über PCR oder LCR durchgeführt werden. Dabei werden Primer verwendet, die die erfindungsgemäße Sequenz oder geeignete Teilbereiche flankieren. Diagnostisch von Bedeutung sind dabei Amplifikationsprodukte von DNA aus dem fraglichen Gewebe, die sich, hinsichtlich des Auftretens von CTAGE-1 oder CTAGE-2 spezifischen Banden von den Amplifikationsprodukten von DNA aus gesundem Gewebe unterscheiden.

In einer alternativen Ausführungsform kann ein Verfahren angewendet werden, das folgende Schritte umfaßt:
- Isolierung von RNA aus dem Patienten,
- Durchführung einer Northern-Analyse mit einer oder mehreren geeigneten Sonden,
- Vergleich der Konzentration und/oder Länge der CTAGE-1 oder CTAGE-2 mRNA der Patientenprobe mit einer mRNA einer gesunden Person, wobei das Vorkommen von CTAGE-1 oder CTAGE-2 mRNA im Vergleich zur Kontroll-mRNA aus Normalgewebe indikativ für eine Tumorerkrankung ist.

Bei diesem Verfahren können dem Fachmann bekannte Methoden bezüglich der Präparation von Gesamt-RNA bzw. poly(A)+RNA aus biologischen Proben, der Auftrennung der RNAs auf nach Größe auftrennenden Gelen, beispielsweise denaturierenden Agarosegelen, der Herstellung und Markierung der Sonde und des Nachweises über "Northern-Blot" angewandt werden.

In einer weiteren alternativen Ausführungsform kann eine mögliche Erkrankung auch durch ein Verfahren diagnostiziert werden, das folgende Schritte umfaßt:
- Gewinnung einer Zellprobe von dem Patienten,
- Inkontaktbringen der so erhaltenen Zellprobe mit dem vorstehend beschriebenen erfindungsgemäßen Antikörper als Sonde unter Bedingungen, die die Bindung des Antikörpers erlauben, wobei eine Bindung des Antikörpers auf eine vorhandene Expression von CTAGE-1 oder CTAGE-2 hinweist, was indikativ für eine Tumorerkrankung ist.

Dieser Nachweis kann ebenfalls unter Anwendung von dem Fachmann bekannten Standardtechniken durchgeführt werden. Diesem sind auch Zellaufschlußverfahren bekannt, die die Isolierung des Proteins auf eine solche Weise erlauben, daß dieses mit dem Antikörper in Kontakt gebracht werden kann. Der Nachweis des gebundenen Antikörpers erfolgt vorzugsweise über Immunassays, beispielsweise Western-Blot, ELISA, FACS oder RIA oder immunhistochemische Verfahren. Weiter eignen sich die Antikörper dafür, um in Tumoren überexprimiertes CTAGE-1 oder CTAGE-2 abzufangen und so das Tumorwachstum zu hemmen, da es Hinweise darauf gibt, daß das Vorkommen von CTAGE-1 oder CTAGE-2 nicht nur das Vorhandensein von Tumoren indikativ anzeigt, sondern aktiv das Tumorwachstum fördert.

Weiter betrifft die vorliegende Erfindung Kits zur Durchführung der erfindungsgemäßen Diagnoseverfahren, die den erfindungsgemäßen Antikörper oder ein Fragment davon, ein erfindungsgemäßes DNA-Molekül als Sonde oder ein für PCR oder LCR geeignetes, auf der Sequenz des erfindungsgemäßen DNA-Moleküls basierendes Primerpaar enthalten, gegebenenfalls in Kombination mit einem geeigneten Nachweismittel.

Je nach Ausgestaltung des mit den erfindungsgemäßen Kits durchzuführenden Diagnoseverfahrens können die in dem Kit enthaltenden DNA-Moleküle, Antikörper oder Fragmente davon an einem geeigneten Träger immobilisiert sein.

Die Erfindung wird nun weiter anhand der Figuren beschrieben, welche zeigen:
- Fig. 1:: Nucleinsäuresequenz der cDNA von CTAGE-1
- Fig. 2:: Nucleinsäuresequenz der cDNA von CTAGE-2
- Fig. 3:: Aminosäuresequenz von CTAGE-2
- Fig. 4:: SEREX-Methode

Die Erfindung wird nun nachfolgend mit Bezug auf die Beispiele beschrieben. Hinsichtlich der verwendeten Methoden wird auf Sambrook, J, Fritsch, E,F. und Maniatis, T. (Molecular cloning; a laboratory manual; second edition; Cold Spring Habor Laboratory Press, 1989) und Current Protocols in Molecular Biology (John Wiley and Sons, 1994-1998) hingewiesen, wobei die nachfolgend erwähnten Techniken, insbesondere Screenen von cDNA-Bibliotheken, Präparation von DNA bzw. RNA, PCR oder Northern Blot dem Fachmann hinreichend bekannt sind und beherrscht werden.

### Beispiel 1:

### Identifizierung von CTAGE-1 aus einer cDNA-Bank mittels SEREX-Methode

Eine cDNA-Phagenbank (in Lambda ZAP) wurde aus mRNA einer gesunden Testis hergestellt (Uni-ZAP™ XR Custom cDNA Library, Fa. Stratagene, Cat # 837201). Die Bank wurde mit der sog. SEREX-Methode durchsucht (Sahin et al., Proc. Natl. Acad. Sci. USA, Vol. 92, S. 11810-11813, 1995). Die Analyse von ca. 1,8 × 10⁶ rekombinanten Klonen dieser Bank mit Seren von Patienten mit kutanen T-Zell-Lymphomen (Mycosis fungoides, Sézary-Syndrom) erbrachte 26 positive Klone. In der serologischen Analyse der gefundenen Klone wurden insgesamt bis zu 11 Seren von erkrankten Patienten und 10 Seren von Kontrollpersonen eingesetzt. Interessanterweise wurde keiner der Klone mit Kontrollseren (aus gesunden Personen) erkannt, wohl aber mit mehreren Seren von erkrankten Patienten. Die Klone wurden sequenziert und die in Figur 1 gezeigte Sequenz für einen der Klone ermittelt. Dieser erhielt die Bezeichnungen CTAGE-1.

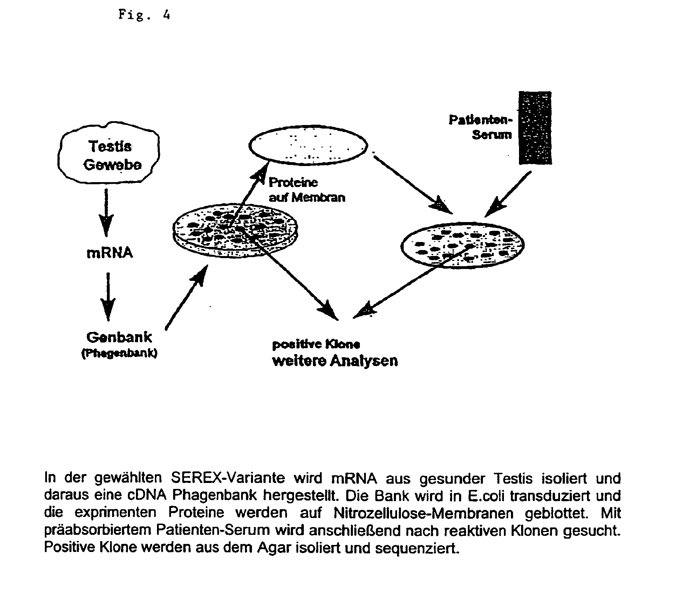

Das erfindungsgemäße Vorgehen ist in der beiliegenden Fig. 4 gezeigt.

### Beispiel 2:

### Herstellung und Reinigung eines erfindungsgemäßen CTAGE-1-Proteins

Zur Herstellung eines erfindungsgemäßen CTAGE-1-Proteins wird die DNA von Fig. 1 mit BamHI-Linkern versehen, mit BamHI nachgeschnitten und in den mit BamHI gespaltenen Expressionsvektor pQE-8 (Qiagen) inseriert. Es wird das Expressionsplasmid pQ/CTAGE-1 erhalten. Ein solches kodiert für ein Fusionsprotein aus 6 Histidin-Resten (N-Terminuspartner) und dem erfindungsgemäßen CTAGE-1-Protein (C-Terminuspartner). pQ/CTAGE-1 wird zur Transformation von E.coli SG 13009 (vgl. Gottesmann, S. et al., J. Bacteriol. 148, (1981), 265-273) verwendet. Die Bakterien werden in einem LB-Medium mit 10 *µ*g/ml Ampicillin und 25 *µ*g/ml Kanamycin kultiviert und 4 h mit 60 *µ*M Isopropyl-β-D-Thiogalactopyranosid (IPTG) induziert. Durch Zugabe von 6 M Guanidinhydrochlorid wird eine Lyse der Bakterien erreicht, anschließend wird mit dem Lysat eine Chromatographie (Ni-NTA-Resin) in Gegenwart von 8 M Harnstoff entsprechend der Angaben des Herstellers (Qiagen) durchgeführt. Das gebundene Fusionsprotein wird in einem Puffer mit pH 3,5 eluiert. Nach seiner Neutralisierung wird das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigt sich, daß ein erfindungsgemäßes (Fusions)protein in hochreiner Form hergestellt werden kann.

### Beispiel 3:

### Herstellung und Nachweis eines erfindungsgemäßen Antikörpers

Ein erfindungsgemäßes Fusionsprotein von Beispiel 2 wird einer 18 % SDS-Polyacrylamid-Gelelektrophorese unterzogen. Nach Anfärbung des Gels mit 4 M Natriumacetat wird die entsprechende Bande aus dem Gel herausgeschnitten und in Phosphat gepufferter Kochsalzlösung inkubiert. Gel-Stücke werden sedimentiert, bevor die Proteinkonzentration des Überstandes durch eine SDS-Polyacrylamid-Gelelektrophorese, dereine Coomassie-Blau-Färbung folgt, bestimmtwird. Mit dem Gel-gereinigten Fusionsportein werden Tiere wie folgt immunisiert:

### Immunisierungsprotokoll für polyklonale Antikörper im Kaninchen

Pro Immunisierung werden 35 *µ*g Gel-gereinigtes Fusionsprotein in 0,7 ml PBS und 0,7 ml komplettem bzw. inkomplettem Freund's Adjuvans eingesetzt.
- Tag 0:: 1. Immunisierung (komplettes Freund's Adjuvans)
- Tag 14:: 2. Immunisierung (inkomplettes Freund's Adjuvans; icFA)
- Tag 28:: 3. Immunisierung (icFA)
- Tag 56:: 4. Immunisierung (icFA)
- Tag 80:: Ausbluten

Das Serum des Kaninchens wird im Immunoblot getestet. Hierzu wird ein erfindungsgemäßes Fusionsprotein von Beispiel 2 einer SDS-Polyacrylamid-Gelelektrophorese unterzogen und auf ein Nitrocellulosefilter übertragen (vgl. Khyse-Andersen), J., J.Biochem.Biophys. Meth. 10, (1984), 203-209). Die Western Blot-Analyse wird wie in Bock, C.-T. et al., Virus Genes 8, (1994), 215-229, beschrieben, durchgeführt. Hierzu wird das Nitrocellulosefilter 1 h bei 37°C mit einem ersten Antikörper inkubiert. Dieser Antikörper ist das Serum des Kaninchens (1:10000 in PBS). Nach mehreren Waschschritten mit PBS wird das Nitrocellulosefilter mit einem zweiten Antikörper inkubiert. Dieser Antikörper ist ein mit alkalischer Phosphatase gekoppelter monoklonaler Ziege Anti-Kaninchen-lgG-Antikörper (Dianova) (1:5000) in PBS. Nach 30-minütiger Inkubation bei 37°C folgen mehrere Waschschritte mit PBS und anschließend die alkalische Phosphatase-Nachweisreaktion mit Entwicklerlösung (36 *µ*m 5' Bromo-4-chloro-3-indolylphosphat, 400 *µ*M Nitroblau-tetrazolium, 100 mM Tris-HCI, pH 9,5, 100 mM NaCl, 5 mM MgCl₂) bei Raumtemperatur, bis Banden sichtbar werden.

Es zeigt sich, daß erfindungsgemäße, polyklonale Antikörper hergestellt werden können.

### Immunisierungsprotokoll für polyklonale Antikörper im Huhn

Pro Immunisierung werden 40 *µ*g Gel-gereinigtes Fusionsprotein in 0,8 ml PBS und 0,8 ml kompletten bzw. inkomplettem Freund's Adjuvans eingesetzt.
- Tag 0:: 1. Immunisierung (komplettes Freund's Adjuvans)
- Tag 28:: 2. Immunisierung (inkomplettes Freund's Adjuvans; icFA)
- Tag 50:: 3. Immunisierung (icFA).

Aus Eigelb werden Antikörper extrahiert und im Western Blot getestet. Es werden erfindungsgemäße polyklonale Antikörper nachgewiesen.

### Immunisierungsprotokoll für monoklonale Antikörper der Maus

Pro Immunisierung werden 12 µg Gel-gereinigtes Fusionsprotein in 0,25 ml PBS und 0,25 ml komplettem bzw. inkompletten Freund's Adjuvans eingesetzt; bei der 4. Immunisierung ist das Fusionsprotein in 0,5 ml (ohne Adjuvans) gelöst.
- Tag 0:: 1. Immunisierung (komplettes Freund's Adjuvans)
- Tag 28:: 2. Immunisierung (inkomplettes Freund's Adjuvans; icFA)
- Tag 56:: 3. Immunisierung (icFA)
- Tag 84:: 4. Immunisierung (PBS)
- Tag 87:: Fusion

Überstände von Hybridomen werden im Western Blot getestet. Erfindungsgemäße, monoklonale Antikörper werden nachgewiesen.

### Beispiel 4

### Northern Blots

Northern-Blots mit mRNAs aus verschiedenen gesunden Geweben wurden bei 50°C über Nacht in einem Hybridisierungspuffer mit 2xSSC, 10-fach Denhardt-Lösung, 10% Dextransulfat, 1% SDS, 3% SSC und 0,1 mg/ml Lachssperma-DNA sowie der spezifischen Sonde hybridisiert. Die spezifische Sonde wurde durch radioaktives Markieren (random-primed DNA-labelling kit; Roche-Diagnostics) des PCR-Produkts aus einer PCR an Testis-Kontroll-cDNA, wie nachfolgend unter Beispiel 5 beschrieben, hergestellt. Die Filter wurden je einmal bei 65 °C in 2xSSC/-0,1% SDS und in 0,2 × SSC/0,1% SDS gewaschen und mit diesen bei -70 °C ein Kodak-Film oder Fuji-Film 24 bis 7 Tage belichtet.

Der Northern Blots zeigte, daß CTAGE-1 nur in Testis, nicht aber in Kontrollgewebe nachweisbar ist.

### Beispiel 5

### Diagnose von Tumoren mittels PCR

Aus verschiedenen Geweben und Blut wurde mRNA unter Verwendung des "RNAclean"-Kits (Fa. AGS-Hybaid, Heidelberg) nach Vorschriften des Herstellers gewonnen. Die RNA wurde mit DNAse behandelt und mit Hilfe des cDNA-Kits von Roche Diagnostics wurde eine cDNA hergestellt. Dazu wird 1 µg RNA und 20 U AMV Reverse Transkriptase unter Zugabe von 1,6 µg RNAse Inhibitor in einem Gesamtvolumen von 20 µl eingesetzt. Die Vorgehensweise erfolgte nach den Angaben des Herstellers. Die aus der RNA gewonnene cDNA wurde mit Hilfe der Polymerase Ketten Reaktion mittels CTAGE-1 spezifischer Primer auf das Vorhandensein von CTAGE-1 überprüft. Die PCR-Bedingungen waren:
5 *µ*l (ca. 100 ng) zu amplifiziemde cDNA
16 mM (NH₄)₂ SO₄
67 mM Tris-HCI, pH 8,8
1,5 mM MgCl₂
0,01 % Tween 20
200 µM dGTP
200 µM dCTP
200 µM dTTP
200 µM dATP
1 U Taq-DNA-Polymerase pro Ansatz (= 25 µl)
400 nM Primer 1
400 nM Primer 2
10 % DMSO

95°C 5 min.
für 35 Zyklen: 95°C 1 Min., 60°C 1 Min., 72°C 2 Min.
72°C 10 Min.
Primer 1: 5'-CTC CTG ACT TCT TTC CCA ACC TTT ACC-3'
Primer 2: 5'-TGC AAT TCC CAC CTA ACT TCC ATT CTG-3

Die Amplifikate wurden nach der elektrophoretischen Auftrennung auf einem Agarosegel (1,5%) durch Ethidiumbromid sichtbar gemacht.

Die Ergebnisse sind wie folgt:

### Kontrollgewebe:

Bei Verwendung von cDNA aus Testis Gewebe, war die PCR unter Verwendung der angegebenen Primem positiv, während alle nachfolgend aufgeführten Gewebe ein negatives Ergebnis erbrachten:

Darm, Dünndarm, fetale Leber, fetale Lunge, fetale Milz, fetale Niere, fetale Skelett Muskel, fetaler Thymus, fetales Gehirn, fetales Herz, Gehirn, Haut, Herz, Knochen, Leber, Lunge, Magen, Milz, Niere, Ovar, Pankreas, Periphere Blut Lymphozyten, Plazenta, Prostata, Skelett Muskel, T-Lymphozyten (aktiviert), Thymus, Trachea.

| *Tumorgewebe (kutanes T-Zell Lymphom; 6 von 17 positiv = 35%):* | |
|---|---|
| Mykosis fungoides: | 4 von 13 positiv |
| T-Zonen Lymphom: | 1 von 1 positiv |
| Sézary Syndrom: | 1 von 2 positiv |
| CD3O-Lymphom; | 0 von 1 positiv |

| *Tumorgewebe von Ovarial-Tumoren (19 von 59 positiv = 32%):* | |
|---|---|
| Tumor | 17 von 59 positiv |
| Rezidiv | 2 von 4 positiv |

| *Tumorgewebe von Tumoren aus dem HNO-Bereich (5 von 27 positiv = 19%):* | |
|---|---|
| Gehörgang | 0 von 1 positiv |
| Hypopharynx | 1 von 3 positiv |
| | |
| Karzinosarcom Parotis | 0 von 1 positiv |
| Larynx | 2 von 12 positiv |
| Nasenrachen | 0 von 1 positiv |
| Orbita | 0 von 1 positiv |
| Oropharynx | 2 von 8 positiv |

| *Zell-Linien:* | |
|---|---|
| Lymphom Zell-Linien: | 4 von 4 positiv (100%) |
| Leukämie Zell-Linien: | 0 von 6 positiv (0%) |
| Melanom Zell-Linien: | 0 von 20 positiv (0%) |

### Beispiel 6: Identifizierung von CTAGE-2

Die Identifizierung von CTAGE-2 erfolgte mittels Race PCR (Fa. Clontech, Heidelberg).
1. Kits / Reagenzien
SMART™ cDNA Amplification Kit
Advantage ^{R} 2 Polymerase PCR Kit
MMLV reverse Transkriptase (Fa. Gibco, Cat. #18064-014)

2. Herstellung von SMART™ cDNA
5'RACE Ready cDNA
   1 *µ*g RNA (Testis)
   1 *µ*l 5' CDS Primer
   1 *µ*l SMART II Oligo
3' RACE Ready cDNA
   1 *µ*g RNA (Testis)
   1 *µ*l 3' CDS Primer
auffüllen auf jeweils 5*µ*l Volumen mit H₂O
   2 min bei 72°C inkubieren
   auf Eis für 2 min
   anzentrifugieren
2 µl 5x First Strand Puffer
   1 µl DTT (20mM)
   1 µl dNTP Mix (10mM)
   1 µl MMLV reverse Transkriptase (200 U/µl)
Mischen durch Pipettieren
   anzentrifugieren
   1,5 h bei 42°C inkubieren
   Stop der Reaktion mit Tricina EDTA Puffer:
   20 µl bei RNA < 200ng
   100 µl bei RNA > 200ng
   250 µl bei mRNA
inkubieren 7 min 72°C
   aufbewahren bei -20°C

3. Primerwahl
Die Primer sollten ca. 23-28 Nukleotide lang sein und mit einer sehr hohen Annealing Temperatur (am besten über 70°C) gewählt werden. Der in 5'Richtung verlängernde Primer, sollte in Nähe des 5' Endes, der 3' verlängernde in Nähe des 3' Endes liegen. Die Primerwahl liegt im Wissen des Fachmanns.
Der 5' verlängernde Primer ist ein reverse Primer und wird als Race 1 bezeichnet, der 3' verlängernde Primer ist ein forward Primer und wird als Race 2 bezeichnet.
4. Race PCR
4.1 Vorbereitung Master Mix herstellen

| | | |
|---|---|---|
| PCR Grade Water | 34,5 *µ*l | 17,25 *µ*l |
| 10x Advantage 2 Puffer | 5 *µ*l | 2,5 *µ*l |
| dNTP Mix | 1 µl | 0,5 µl |
| 50x Advantage 2 Poly | 1 µl | 0,5 µl |

| | | |
|---|---|---|
| vortexen, anzentrifugieren | | |

Kontroll PCR 1:

| | | | |
|---|---|---|---|
| 5' cDNA | 1,25 µl | 3' Race cDNA | 1.25 µl |
| UPM 10× | 2,5 µl | UPM 10× | 2,5 µl |
| 5' Race TFR Primer | 0,5 µl | 3' Race TFR Primer | 0,5 µl |
| H₂O | - | H₂O | - |
| Master Mix | 20,75 *µ*l | Master Mix | 20,75 *µ*l |

Touch Down PCR

| | | |
|---|---|---|
| 94°C, 5 min | | |
| 94°C, 0,3 min 5x; | 94°C, 0,3 min 5x; | 94°C, 0,3 min 25x |
| 70°C, 0,3 min 5x; | 68°C, 0,3 min5x; | 66°C, 0,3 min 25x |
| 72°C, 3 min 5x; | 72°C, 3 min 5x; | 72°C, 3 min 25x |
| 72°C, 10 min | | |
| 4°C, aufbewahren | | |

erwartetes Ergebnis:
5': 2,6 kb
3': 2,9 kb (manchmal 0,6 kb)
Ergebnis: cDNA in Ordnung
4.2 Durchführung Race PCR
Pipetierschema:

| 5 ' Richtung: | | | |
|---|---|---|---|
| | Race | negativ Kontrolle | negativ Kontrolle |
| 5'cDNA | 2,5 µl | 1,25 µl | 1,25 µl |
| UPM 10× | 5 µl | 2,5 µl | 2,5 µl |
| Race 1 Primer | 1 µl | - | 0,5 µl |
| Kontrollprimer: TFR 5' | - | 0,5 | - |
| H₂O | - | - | - |
| Master Mix | 41,5 µl | 20,75 µl | 20,75 µl |

| 3' Richtung: | | | |
|---|---|---|---|
| | Race | negativ Kontrolle | negativ Kontrolle |
| 3'cDNA | 2,5 µl | 1,25 µl | 1,25 µl |
| UPM 10× | 5 µl | - | 2,5 µl |
| Race 1 Primer | 1 µl | - | - |
| Kontrollprimer: TFR 5' | - | 0,5 µl | - |
| H₂O | - | 2,5 µl | 0,5 µl |
| Master Mix | 41,5 µl | 20,75 µl | 20,75 µl |

Programm: Touch Down PCR

| | | |
|---|---|---|
| 94°C, 5 min | | |
| 94°C, 0,3 min 5x; | 94°C, 0,3 min 5x; | 94°C, 0,3 min 25x |
| 70°C, 0,3 min 5x; | 68°C, 0,3 min 5x; | 66°C, 0,3 min 25x |
| 72°C, 3 min 5x; | 72°C, 3 min 5x; | 72°C, 3 min 25x |
| 72°C, 10 min | | |
| 4°C, aufbewahren | | |

Ergebnis:
Negativkontrollen: keine Amplifikation
Race: Amplifikation hat stattgefunden
Alternativen, wenn Race nur "Schmier" liefert: nested Race
Dafür PCR Amplifikat von 1. Race 1:50 in Tris EDTA Puffer verdünnen
nested Race:
verändertes Pipettierschema: statt 5 *µ*l UPM, 1 *µ*l NUP (nested Universal Primer Mix)
statt Race 1, nested Race β
verändertes PCR Programm: statt Touch Down PCR, 15 Zyklen nes Race
94 °C, 5 min
94 °C, 0,3 min 15x
68°C, 0,3min 15x
72°C, 3min 15x
72°C, 10min
4°C, aufbewahren
Mit dem beschriebenen Vorgehen erfolgt eine schnelle Amplifikation der cDNA Enden:
5.1 cDNA Synthese
5.1.1. 3'
   3'cDNA Synthese nutzt eine normale reverse Transkriptase Reaktion mit einem initialem Oligo dT Primer, der am Poly A Ende hybridisiert.
   Im 3' CDS (dT Oligomix) Mix ist auch das Smart Oligoll (in geringere Konzentration) enthalten (siehe 5.2.2), so daß ein 5' full length Polymerisierung stattfindet. Am Oligo dT Primer ist eine bekannte Sequenz angehängt (= Smart).
**5.1.2. 5'**
   5'cDNA Synthese wird, wie die 3' Synthese, durch ein Oligo dT (5'CDS Mix) am 3' Ende der mRNA gestartet.
   Das Smart II Oligo System nutzt die Besonderheit aus, daß die Reverse Transkriptase MMLV, nach einer full length Transkription, eine terminal Transferase Aktivität übernimmt, die 3-5 Nukleotide (vorrangig dCtp) am neu synthetisierten Strang (1^{st} strand) anhängt.
   An dieser Cytosin reichen Region kann das SMART II Oligo binden, da es eine Guanin reiche Region besitzt. An dieser ist eine bekannte Sequenz (Smart II Oligo) drangehängt. Das SMART II Oligo dient als Primer für die reverse Transkription jetzt in 3' Richtung.
   Die MMLV Transkriptase hängt nur an voll durch polymerisierte mRNAs eine solche cytosinreiche reiche Region, so daß dadurch gewährleistet wird, das nur full length cDNA entstehen.

5.2. Race PCR:
**5.2.1 Advantage 2 Polymerase Mix**
   Advantage Taq™ Polymerase
   Proof reading Polymerase
   Taq Start™ Antibody
   Die Proof Reading bewirkt eine fehlerfrei Polymerisierung.
   Der Antikörper bewirkt eine automatische hot start PCR. Der monoklonale Antikörper bindet am N Terminus der Taq Polymerase und inhibiert somit seine Aktivität. Beim initialen 95°C Erhitzen wird der Antikörper denaturiert und die Polymerase wird nicht mehr inhibiert.
   Alles in allem ermöglicht diese Kombination eine Polymerase Reaktion über lange Distanzen.
**5.2.2 PCR**
   Die Race PCR nutzt aus, daß die SMART full length cDNA durch bekannte Sequnzen begrenzt ist. Diese dienen als Forward / reverse Primer für die Amplifikation Die bekannte Sequenz, die am Oligo dT im 3' CDS Primer Mix angehängt ist, ist die gleiche, die das SMART Oligo II Anhängsel an die Guanin reichen Sequenz darstellt.

## Patentansprüche

1. Nukleinsäure, die für ein tumor-assoziiertes Antigen kodiert, wobei die Nukleinsäure besteht aus:
(a) der Nukleinsäure von Fig. 1 oder Fig. 2 oder einer dazu mindestens 80% identischen Nukleinsäure,
(b) einer mit der Nukleinsäure von (a) über den degenerierten genetischen Code verwandte Nukleinsäure.

2. Nukleinsäure nach Anspruch 1, wobei sie eine cDNA oder genomische DNA ist.

3. Vektor, umfassend die Nukleinsäure nach Anspruch 1.

4. Vektor nach Anspruch 3, wobei die Nukleinsäure mit regulatorischen Elementen funktionell verknüpft ist, die die Expression in prokaryontischen oder eukaryontischen Wirtszellen erlauben.

5. Vektor nach Anspruch 3 oder 4, der ein Plasmid, Bakterium, Virus oder Retrovirus ist.

6. Transformante, enthaltend den Vektor nach Anspruch 3, 4 oder 5.

7. Transformante nach Anspruch 6, die ein Bakterium, eine Hefe-, Insekten-, Tier-oder Säugerzelle ist.

8. Tumor-assoziiertes Antigen, welches von der Nukleotidsequenz von Fig. 1 oder einer dazu mindestens 80% identischen Nukleotidsequenz kodiert wird.

9. Tumor-assoziiertes Antigen, welches die Aminosäuresequenz von Fig. 3 oder eine dazu mindestens 80% identische Aminosäuresequenz umfasst.

10. Verfahren zur Herstellung des Antigen nach Anspruch 8 oder 9, umfassend die Kultivierung der Transformante nach Anspruch 6 oder 7 unter Bedingungen, die die Expression des Proteins erlauben, und Gewinnung des Proteins aus der Kultur.

11. Antikörper, der das Antigen nach Anspruch 8 oder 9 spezifisch erkennt.

12. Verwendung (a) der Aminosäuresequenz, die der Nukleotidsequenz gemäß Fig 1. abgeleitet ist oder einer dazu mindestens 80% identischen Aminosäurensequenz oder (b) der Aminosäuresequenz gemäß Fig. 3 oder einer dazu mindestens 80% identischen Aminosäurensequenz zur Herstellung eines tumor-spezifischen Antigens zur Diagnose und/oder Therapie von Tumoren.

13. Nukleinsäure nach Anspruch 1 oder 2 zur Verwendung als Reagens zur Diagnose und/oder Therapie von Tumoren.

14. Spezifischer Antikörper nach Anspruch 11 zur Verwendung als Reagens zur Diagnose und/oder Therapie von Tumoren.

15. Verwendung eines Antigen nach Anspruch 12, einer Nukleinsäure nach Anspruch 13 oder eines Antikörpers nach Anspruch 14 zur Herstellung eines Vakzinierungsmittels für die Therapie von Tumoren.

16. Verwendung eines Antigen nach Anspruch 12 zur Erzeugung Peptidbeladener Antigen-präsentierender Zellen.

17. Verwendung eines Antigen nach Anspruch 12 oder einer Nukleinsäure nach Anspruch 13 zur Erzeugung tumor-spezifischer zytotoxischer Zellen.

18. Verwendung nach einem der Ansprüche 15 bis 17, wobei das Antigen ein HLA-abhängiges Peptid-Fragment ist.

## Claims

1. A nucleic acid which codes for a tumor-associated antigen, wherein the nucleic acid consists of:
(a) the nucleic acid of figure 1 or figure 2 or a nucleic acid at least 80 % identical therewith,
(b) a nucleic acid related to the nucleic acid of (a) via the degenerated genetic code.

2. The nucleic acid according to claim 1, which is a cDNA or genomic DNA.

3. A vector comprising the nucleic acid according to claim 1.

4. The vector according to claim 3, wherein the nucleic acid is functionally linked with regulatory elements which enable the expression in prokaryotic or eukaryotic host cells.

5. The vector according to claim 3 or 4, which is a plasmid, bacterium, virus or retrovirus.

6. A transformant, containing the vector according to claim 3, 4 or 5.

7. The transformant according to claim 6, which is a bacterium, a yeast cell, insect cell, animal or mammalian cell.

8. A tumor-associated antigen which is encoded by the nucleotide sequence of figure 1 or a nucleotide sequence at least 80 % identical therewith.

9. The tumor-associated antigen which comprises the amino acid sequence of figure 3 or an amino acid sequence at least 80 % identical therewith.

10. A method of preparing the antigen according to claim 8 or 9, comprising culturing the transformant according to claim 6 or 7 under conditions enabling the expression of the protein and collecting the protein from the culture.

11. An antibody, which specifically detects the antigen according to claim 8 or 9.

12. Use of (a) the amino acid sequence derived from the nucleotide sequence according to figure 1 or an amino acid sequence at least 80 % identical therewith or (b) the amino acid sequence according to figure 3 or an amino acid sequence at least 80 % identical therewith for the production of a tumor-specific antigen for the diagnosis and/or treatment of tumors.

13. Nucleic acid according to claim 1 or 2 for use as a reagent for the diagnosis and/or treatment of tumors.

14. Specific antibody according to claim 11 for use as a reagent for the diagnosis and/or treatment of tumors.

15. Use of an antigen according to claim 12, of a nucleic acid according to claim 13, or an antibody according to claim 14 for preparing a vaccination agent for the treatment of tumors.

16. Use of an antigen according to claim 12 for the production of peptide-loaded antigen-presenting cells.

17. Use of an antigen according to claim 12 or a nucleic acid according to claim 13 for the production of tumor-specific cytotoxic cells.

18. Use according to any of claims 15 to 17, wherein the antigen is a HLA-dependent peptide fragment.

## Revendications

1. Acide nucléique codant un antigène associé à une tumeur où l'acide nucléique consiste en :
(a) l'acide nucléique de la figure 1 ou 2 ou bien un acide nucléique au moins identique au moins à 80% à celui-ci,
(b) un acide nucléique utilisé avec l'acide nucléique de (a) avec le code génétique dégénéré.

2. Acide nucléique selon la revendication 1, **caractérisé en ce qu'**il est constitué par un cADN ou bien par un ADN génomique.

3. Vecteur, **caractérisé en ce qu'**il comprend l'acide nucléique selon la revendication 1.

4. Vecteur selon la revendication 3, **caractérisé en ce que** l'acide nucléique est lié fonctionnellement avec des éléments de régulation, qui permettent l'expression des cellules hôtes procariotiques ou eucariotiques.

5. Vecteur selon la revendication 3 ou 4, **caractérisé qu'**il est constitué par un plasmide, une bactérie, un virus ou un rétrovirus.

6. Organisme transformé comprenant le vecteur selon la revendication 3, 4 ou 5.

7. Organisme transformé selon la revendication 6, **caractérisé en ce qu'**il est constitué par une bactérie, une cellule de levure, d'insecte, d'animal ou de mammifère.

8. Antigène associé à une tumeur, qui est codé par une séquence nucléotide selon la figure 1 ou bien une séquence nucléotide identique au moins à 80% de celle-ci.

9. Antigène associé à une tumeur, qui comprend la séquence acide aminé de la figure 3 ou bien une séquence acide aminé identique au moins à 80% à celle-ci.

10. Procédé de préparation d'un antigène selon la revendication 8 ou 9, **caractérisé en ce qu'**il comprend la culture d'un organisme transformé selon la revendication 6 ou 7 dans des conditions qui autorisent l'expression de la protéine et la récupération de la protéine à partir de la culture.

11. Anticorps, **caractérisé en ce qu'**il reconnaît spécifiquement l'antigène selon la revendication 8 ou 9.

12. Utilisation (a) de la séquence d'acide aminé, qui est orientée par la séquence nucléotide selon la figure 1, ou bien d'une séquence acide aminé identique au moins à 80% de celle-ci, ou bien (b) de la séquence de l'acide aminé selon la figure 3 ou de la séquence de l'acide aminé identique au moins à 80% de celle-ci à la préparation d'un antigène spécifique à une tumeur pour le diagnostique et/ou le traitement des tumeurs.

13. Acide nucléique selon la revendication 1 ou 2 pour l'utilisation comme réactif pour le diagnostique et/ou le traitement des tumeurs.

14. Anticorps spécifique selon la revendication 11 pour l'utilisation comme réactif pour le diagnostique et/ou le traitement des tumeurs.

15. Utilisation d'un antigène selon la revendication 12, d'un acide aminé selon la revendication 13 ou bien d'un anticorps selon la revendication 14 dans la préparation d'un moyen de vaccination pour le traitement des tumeurs.

16. Utilisation d'un antigène selon la revendication 12 pour la production de cellules présentant des antigènes chargés en peptide.

17. Utilisation d'un antigène selon la revendication 12 ou bien d'un acide nucléique selon la revendication 13 pour la production de cellules cytotoxiques spécifiques aux tumeurs.

18. Utilisation selon l'une des revendications 15 à 17, dans laquelle l'antigène est un fragment peptidique indépendant de HLA.
